# DEMANDE DE BREVET EUROPEEN

(11) **EP 1 348 424 A1**
(43) Date de publication de la demande: **01.10.2003**
(21) Numéro de dépôt: 03290564.8
(22) Date de dépôt: 07.03.2003
(51) Int. Cl.: A61K 7/48, A61K 7/42

(54) **Composition autobronzante contenant un tetrahydrocurcuminoide et un agent autobronzant**

(30) Priorité: 28.03.2002 FR 0203934
(71) Demandeur: L'OREAL, 75008 Paris (FR)
(72) Inventeur: Candau, Didier, 91570 Bievres (FR)
(74) Mandataire: Miszputen, Laurent

(57) **Abrégé**

La présente invention se rapporte à une composition cosmétique et/ou dermatologique plus particulièrement destinée au bronzage et/ou au brunissage artificiels de la peau et comprenant, dans un support cosmétiquement acceptable, au moins mélange de dérivés de la 1,7-bisphényl heptane-3,5-dione de structure particulière et au moins un agent autobronzant.

La présente invention se rapporte encore à un procédé de traitement cosmétique pour bronzer ou brunir artificiellement la peau et à l'utilisation d'au moins un dérivé ou un mélange de dérivés de la 1,7-bisphényl heptane-3,5-dione de structure particulière pour améliorer la coloration et/ou la stabilité d'un agent autobronzant.

L'invention concerne également les applications de ces compositions à la coloration de la peau proche du bronzage naturel de la peau.

## Description

La présente invention se rapporte à une composition cosmétique et/ou dermatologique plus particulièrement destinée au bronzage et/ou au brunissage artificiels de la peau et comprenant, dans un support cosmétiquement acceptable, au moins un dérivé ou un mélange de dérivés de la 1,7-bisphényl heptane-3,5-dione de structure particulière et au moins un agent autobronzant.

La présente invention se rapporte encore à un procédé de traitement cosmétique pour bronzer ou brunir artificiellement la peau et à l'utilisation d'au moins un dérivé ou un mélange de dérivés de la 1,7-bisphényl heptane-3,5-dione de structure particulière pour améliorer la coloration et/ou la stabilité d'un agent autobronzant.

L'invention concerne également les applications de ces compositions à la coloration de la peau proche du bronzage naturel de la peau.

"Par agent autobronzant" au sens de la présente demande, on entend un agent qui, appliqué sur la peau, notamment sur le visage, permet d'obtenir un effet de bronzage d'apparence plus ou moins semblable à celui qui peut résulter d'une exposition prolongée au soleil (bronzage naturel) ou sous une lampe UV.

De nos jours, il est important d'avoir bonne mine et une peau bronzée est toujours signe de bonne santé. Cependant, le bronzage naturel n'est pas toujours souhaitable dans la mesure où il nécessite des expositions prolongées aux rayonnements UV, en particulier aux rayonnements UV-A qui provoquent le brunissement de la peau mais en contrepartie sont susceptibles d'induire des réactions voire une altération de celle-ci, notamment dans le cas d'une peau sensible ou d'une peau continuellement exposée au rayonnement solaire : érythème, brûlure, perte d'élasticité, apparition de rides, vieillissement prématuré. Il est donc souhaitable de trouver une alternative au bronzage naturel qui soit compatible avec les exigences de telles peaux.

La plupart des produits cosmétiques destinés au bronzage artificiel de la peau sont à base de dérivés carbonylés permettant, par interaction avec les acides aminés de la peau, la formation de produits colorés, parmi ceux-ci on compte des composés mono- ou polycarbonylés tels que par exemple l'isatine, l'alloxane, la ninhydrine, le glycéraldéhyde, l'aldéhyde mésotartrique, la glutaraldéhyde, l'érythrulose, la dihydroxyacétone (DHA).

La DHA, est un produit particulièrement intéressant qui est couramment utilisé en cosmétique comme agent de bronzage artificiel de la peau ; appliqué sur cette dernière, notamment sur le visage, il permet d'obtenir un effet de bronzage ou de brunissage d'apparence semblable à celui qui peut résulter d'une exposition prolongée au soleil (bronzage naturel) ou sous une lampe UV.

Un inconvénient de la DHA est la lenteur avec laquelle la coloration se développe : il faut en effet compter plusieurs heures (3 à 5 heures en général) pour que soit révélée la coloration. L'intensité de la coloration obtenue sur la peau et /ou sa tenue au cours du temps (résistance au lavage) et/ou la rapidité avec laquelle la coloration se développe, sont souvent jugées insuffisantes par les utilisateurs de compositions autobronzantes à base de DHA.

Un autre problème posé par les compositions à base de DHA est qu'elles présentent la fâcheuse tendance, plus ou moins prononcée selon la nature du milieu dans lequel elles sont formulées, à se dégrader au cours du temps. Ces problèmes liés au stockage et/ou à la conservation des compositions à base de DHA se traduisent généralement à terme par un jaunissement non souhaitable de ces compositions.

Il existe donc une demande croissante de produits autobronzants agissant rapidement et conférant une coloration proche du bronzage naturel.

De manière surprenante et avantageuse, la demanderesse a trouvé que l'utilisation d'au moins un dérivé ou un mélange de dérivés de la 1,7-bisphényl heptane-3,5-dione de structure particulière de formule (I) telle que définie ci-dessous permettait d'améliorer la stabilité et/ou la coloration des compositions comprenant un agent autobronzant. Les colorations obtenues sont plus chromatiques, plus stables dans le temps et présentent une bonne homogénéité.

La composition selon la présente invention comprend dans un support cosmétiquement acceptable au moins un dérivé ou un mélange de dérivés de la 1,7-bisphényl heptane-3,5-dione de structure particulière de formule (I) telle que définie ci-dessous et au moins un agent autobronzant.

La présente demande a encore pour objet l'utilisation de la composition selon l'invention à titre de composition destinée au bronzage ou au brunissement de la peau ; et un procédé cosmétique de bronzage ou brunissement de la peau tel qu'il consiste à appliquer sur la peau une quantité efficace d'une composition selon l'invention.

Enfin, la présente demande concerne encore l'utilisation d'au moins un dérivé ou un mélange de dérivés de la 1,7-bisphényl heptane-3,5-dione de structure particulière de formule (I) telle que définie ci-dessous dans des compositions pour le bronzage et/ou le brunissage artificiels de la peau contenant au moins un agent autobronzant en vue d'améliorer la coloration et/ou la stabilité dudit agent autobronzant.

D'autres caractéristiques, aspects et avantages de la présente invention apparaîtront à la lecture de la description détaillée qui va suivre.

Les dérivés de la 1,7-bisphényl heptane-3,5-dione conformes à l'invention répondent à la formule (I) suivante : 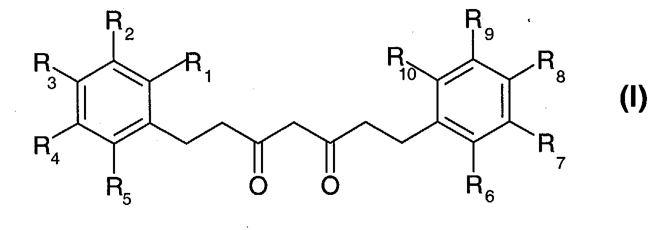 dans laquelle :
R₁ , R₂ , R₃, R₄ , R₅ , R₆, R₇, R₈, R₉, R₁₀, identiques ou différents, sont choisis parmi:
   (i) un atome d'hydrogène ;
   (ii) un radical alkyle linéaire ou ramifié en C₁-C₄ ;
   (iii) un radical OR₁₁ dans lequel R₁₁ est choisi parmi un atome d'hydrogène, un radical alkyle linéaire ou ramifié en C₁-C₄, un radical PO (OX₁)(OX₂) ou un radical SO₂(OX₃) dans lesquels, X₁, X₂, et X₃, identiques ou différents, désignent un atome d'hydrogène ou un cation de métal alcalin ou NH₄⁺ , X₁ et X₂ pouvant également désigner ensemble un cation de métal divalent,
   (iv) un radical acyle R₁₂CO dans lequel R₁₂ est choisi parmi des radicaux hydrocarbonés en C₁-C₃₀, qui sont linéaires ou ramifiés, saturés ou non saturés, hydroxylés ou non hydroxylés, carboxylés ou non carboxylés ;
   (v) un radical glycosyle ou uronyle ;
   lorsqu'un radical R₁ à R₁₀ désigne un reste OR₁₁, il peut également former avec le cycle aromatique auquel il est attaché et un radical adjacent un cycle contenant 5 ou 6 atomes ; étant entendu qu'au moins un des radicaux R₁ à R₁₀ désigne un reste OR₁₁.

Dans la formule (I) décrite ci-dessus, les radicaux alkyle peuvent désigner notamment les radicaux méthyle, éthyle, propyle, isopropyle, n-propyle, butyle, n-butyle, tert-butyle ; préférentiellement le radical alkyle désigne le radical méthyle.

Dans la formule (I) décrite ci-dessus, le radical glycosyle désigne plus particulièrement un radical glucosyle.

Dans la formule (I) décrite ci-dessus, le radical uronyle désigne plus particulièrement un radical mannuronyle ou glucuronyle.

Les composés de formule (I) sont connus en tant que tels. Les tétrahydrocurcuminoides conformes à l'invention sont obtenus par réduction de la curcumine ou synthétisés tels que décrits dans les articles « Synthesis and antibacterial activity of tetrahydrocurcuminoids ; S Venkateswarlu, M Rambabu, GV Subbaraju and S Satyanarayana ; Asian Journal of Chemistry-2000-1-141-144.» , « Synthesis of naturally occuring curcuminoids and related compounds ; U Pedersen, PB Rasmussen, SO Lawesson ; Liebigs Ann. Chem.-1985-1557-1569., les demandes de brevet JP02051595, JP02069431, JP02049747 et JP02128133, le brevet US 5,266,344 et la demande WO00/61162.

Parmi les composés de formule (I) préférentiels selon la présente invention, on peut citer :
- la 1,7-bis-(3-hydroxy-4-méthoxyphényl)heptane-3,5-dione) ou tétrahydrocurcumine (THC) de structure : 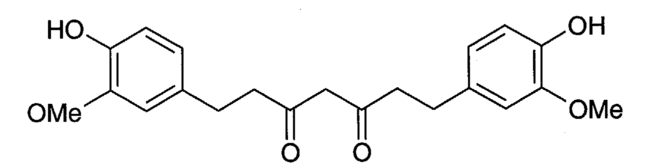
- la 1-(3-méthoxy-4-hydroxy phényl) 7-(4'-hydroxy phényl) heptane-3,5-dione ou tétrahydrodéméthoxycurcumine (THDC) de structure : 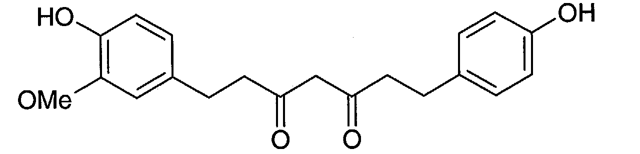
- la 1,7-bis-(4-hydroxy phényl) heptane-3,5-dione ou tétrahydrobis déméthoxycurcumine (THBDC) de structure : 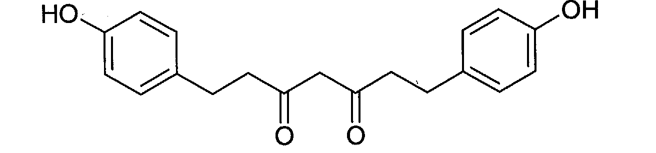
- la 1,7-bis(3,4-dihydroxyphenyl) heptane-3,5-dione de structure : 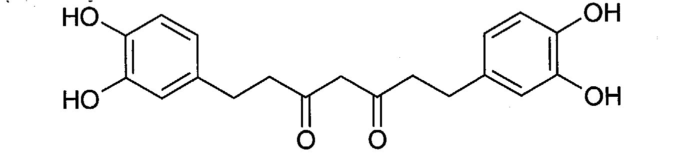 ainsi que leurs mélanges.

Une forme particulière de l'invention consiste à utiliser comme seul composé de formule (I) la 1,7-bis-(3-hydroxy-4-méthoxyphényl)heptane-3,5-dione) ou tétrahydrocurcumine (THC).

Une autre forme particulière de l'invention consiste à utiliser un mélange de composés de formule (I) constitué :
de tétrahydrocurcumine ;
de tétrahydro-déméthoxycurcumine et
de tétrahydro-bis-déméthoxycurcumine.

On utilisera encore plus particulièrement un mélange constitué :
- de 70 à 95% en poids de tétrahydrocurcumine ;
- de 4 à 25% en poids de tétrahydro-déméthoxycurcumine et
- de 0,5 à 10 % en poids de tétrahydro-bis-déméthoxycurcumine

Et encore plus particulièrement un mélange constitué :
- de 75 à 90% en poids de tétrahydrocurcumine ;
- de 8 à 20% en poids de tétrahydro-déméthoxycurcumine et
- de 1 à 5% en poids de tétrahydro-bis-déméthoxycurcumine
comme le produit décrit et synthétisé dans la demande WO00/61162 et fourni par la société SABINSA CORPORATION sous la dénomination commerciale Tetrahydrocurcuminoids CG ou Tetrahydrocurcuminoids.

De façon générale, le ou les composés de formule (I) présents dans la composition sont totalement dissous dans le support de la composition.

Les composés de formule (I) selon l'invention représentent de préférence de 0,001 à 10% en poids environ du poids total de la composition cosmétique, plus particulièrement de 0,01 à 5%, et plus préférentiellement encore de 0,1 à 5% en poids environ de ce poids.

Les agents autobronzants sont généralement choisis parmi les composés mono ou polycarbonylés tels que par exemple l'isatine, l'alloxane, la ninhydrine, le glycéraldéhyde, l'aldéhyde mésotartrique, la glutaraldéhyde, l'érythrulose, les dérivés de pyrazolin-4,5-diones telles que décrites dans la demande de brevet FR 2 466 492 et WO 97/35842, la dihydroxyacétone (DHA), les dérivés de 4,4-dihydroxypyrazolin-5-ones telles que décrites dans la demande de brevet EP 903 342. On utilisera de préférence la DHA.

La DHA peut être utilisée sous forme libre et/ou encapsulée par exemple dans des vésicules lipidiques telle que des liposomes, notamment décrits dans la demande WO 97/25970.

Ces agents autobronzants peuvent être associés à au moins un colorant direct synthétique ou naturel et/ou au moins un dérivé indolique comme ceux décrits dans les brevets EP 425 324 et EP 456 545.

Ces agents autobronzants peuvent également être associés à d'autres agents de coloration de la peau synthétiques ou naturels.

Au sens de la présente invention, on entendra par « agent de coloration de la peau », tout composé ayant une affinité particulière pour la peau lui permettant de conférer à cette dernière une coloration durable, non-couvrante (à savoir n'ayant pas tendance à opacifier la peau) et qui ne s'élimine ni à l'eau ni à l'aide d'un solvant, et qui résiste à la fois au frottement et au lavage par une solution contenant des tensioactifs. Une telle coloration durable se distingue donc de la coloration superficielle et momentanée apportée par exemple par un pigment de maquillage.

Les agents de coloration additionnels peuvent également être choisis par exemple parmi les extraits végétaux comme par exemple les extraits de bois rouges « insolubles » du genre Pterocarpus et du genre Baphia comme le Pterocarpus santalinus, le Pterocarpus osun, le Pterocarpus soyauxii, le Pterocarpus erinaceus, le Pterocarpus indicus ou encore le Baphia nitida comme ceux décrits dans la demande de brevet EP 971 683.

Les agents de coloration additionnels peuvent également être choisis par exemple parmi d'autres extraits végétaux comme par exemple les extraits de sorgho obtenus à partir de la plante entière, des tiges, des graines ou des feuilles du genre Sorghum. Les espèces préférées du Sorghum sont choisies parmi le Sorghum bicolor, le Sorghum caudatum, le Sorghum nervosum, le Sorghum durra, le Sorghum vulgare et les Sorghum en association avec du Colletotrichum Graminicola comme ceux décrits dans la demande de brevet FR0200251.

Les agents de coloration peuvent également être des nanopigments d'oxyde de fer dont la taille moyenne des particules élémentaires est inférieure à 100nm tels que ceux décrits dans la demande de brevet EP 966 953.

Les agents autobronzants sont généralement présents dans les compositions selon l'invention dans des proportions allant de 0,1 à 10% en poids par rapport au poids total de la composition, et de préférence de 0,2 à 8% en poids par rapport au poids total de la composition.

Les compositions autobronzantes conformes à l'invention peuvent se présenter sous forme de crèmes, de laits, de gels, de gel-crèmes, d'émulsions huile-dans-eau, de dispersions vésiculaires, de lotions fluides, en particulier de lotions fluides vaporisables ou tout autre forme généralement utilisée en cosmétique, en particulier celles convenant habituellement aux compositions cosmétiques autobronzantes.

Les compositions conformes à la présente invention peuvent comprendre en outre des adjuvants cosmétiques classiques notamment un adjuvant cosmétique choisis parmi les corps gras, les solvants organiques, les épaississants ioniques ou non ioniques, les adoucissants, les humectants, les opacifiants, les stabilisants, les émollients, les silicones, les agents anti-mousse, les agents répulsifs contre les insectes, les parfums, les conservateurs, les tensioactifs anioniques, cationiques, non-ioniques, zwitterioniques ou amphotères, les antagonistes de substance P, les antagonistes de substance CGRP, les charges, les polymères, les propulseurs, les agents alcalinisants ou acidifiants ou tout autre ingrédient habituellement utilisé dans le domaine cosmétique et/ou dermatologique, en particulier pour la fabrication de compositions autobronzantes sous forme d'émulsions.

Les corps gras peuvent être constitués par une huile ou une cire ou leurs mélanges. Par « huile », on entend un composé liquide à température ambiante. Par « cire », on entend un composé solide ou substantiellement solide à température ambiante, et dont le point de fusion est généralement supérieur à 35 °C.

Comme huiles, on peut citer les huiles minérales (paraffine) ; végétales (huile d'amande douce, de macadamia, de pépin de cassis, de jojoba) ; synthétiques comme le perhydrosqualène, les alcools, les acides ou les esters gras (comme le benzoate d'alcools en C12-C15 vendu sous la dénomination commerciale « Finsolv TN » par la société Finetex, le palmitate d'octyle, le lanolate d'isopropyle, les triglycérides dont ceux des acides caprique/caprylique, les esters et les éthers gras oxyéthylénés ou oxypropylénés ; siliconées (cyclométhicone, polydiméthyl-siloxanes ou PDMS) ou fluorées ; les polyalkylènes et leurs mélanges.

Comme composés cireux, on peut citer la paraffine, la cire de carnauba, la cire d'abeille, l'huile de ricin hydrogénée.

Parmi les solvants organiques, on peut citer les alcools et polyols inférieurs ayant au plus 8 atomes de carbone.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthylcellulose.

Les compositions conformes à l'invention peuvent comporter en plus au moins un agent photoprotecteur organique et/ou au moins un agent photoprotecteur inorganique actifs dans l'UVA et/ou l'UVB (absorbeurs), hydrosolubles ou liposolubles ou bien insolubles dans les solvants cosmétiques couramment utilisés.

Les agents filtrant le rayonnement ultraviolet peuvent être choisis parmi les filtres UV organiques, les agents filtrant les radiations UV minéraux ou leurs mélanges.

Les filtres UV organiques conformes à l'invention peuvent être hydrosolubles, liposolubles ou insolubles dans les solvants usuels cosmétiques. Ils sont choisis notamment parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469 et EP933376 ; les dérivés de la benzophénone notamment ceux décrits dans les demandes EP-A-1046391 et DE10012408 ; les dérivés de β,β'-diphénylacrylate, les dérivés de benzotriazole, les dérivés de benzimidazole ; les imadazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US5,166,355, GB2303549, DE19726184 et EP893119 ; les polymères filtres et silicones filtres tels que ceux décrits notamment dans la demande WO93/04665 ; les dimères dérivés d'α-alkylstyrène tels que ceux décrits dans la demande de brevet DE19855649 ; les dérivés de 4,4-diarylbutadiène tels que ceux décrits dans les demandes de brevet EP0967200 et DE19755649 et EP1133981 ; les hydroxybenzophénones aminosubstituées telles que les structures décrites dans EP1046391 et EP1133980 ; ainsi que leurs mélanges.

Comme exemples de filtres organiques, on peut citer désignés ci-dessus sous leur nom INCI :
Dérivés de l'acide para-aminobenzoigue :
   PABA,
   Ethyl PABA,
   Ethyl Dihydroxypropyl PABA,
   Ethylhexyl Diméthyl PABA vendu notamment sous le nom « ESCALOL 507 » par ISP,
   Glyceryl PABA,
   PEG-25 PABA vendu sous le nom « UVINUL P25 » par BASF,
Dérivés salicyliques :
   Homosalate vendu sous le nom « Eusolex HMS » par Rona/EM Industries,
   Ethylhexyl Salicylate vendu sous le nom « NEO HELIOPAN OS » par Haarmann et REIMER,
   Dipropyleneglycol Salicylate vendu sous le nom « DIPSAL » par SCHER,
   TEA Salicylate, vendu sous le nom « NEO HELIOPAN TS » par Haarmann et REIMER,
Dérivés du dibenzoylméthane :
   Butyl Methoxydibenzoylmethane vendu notamment sous le nom commercial « PARSOL 1789 » par HOFFMANN LA ROCHE,
   Isopropyl Dibenzoylmethane,
Dérivés cinnamiques :
   Ethylhexyl Methoxycinnamate vendu notamment sous le nom commercial « PARSOL MCX » par HOFFMANN LA ROCHE,
   Isopropyl Methoxy cinnamate,
   Isoamyl Methoxy cinnamate vendu sous le nom commercial « NEO HELIOPAN E 1000 » par HAARMANN et REIMER,
   Cinoxate,
   DEA Methoxycinnamate,
   - Diisopropyl Methylcinnamate,
   Glyceryl Ethylhexanoate Dimethoxycinnamate
Dérivés de β,β'-diphénylacrylate :
   Octocrylene vendu notamment sous le nom commercial « UVINUL N539 » par BASF,
   Etocrylene, vendu notamment sous le nom commercial « UVINUL N35 » par BASF,
Dérivés de la benzophénone :
   Benzophenone-1 vendu sous le nom commercial « UVINUL 400 » par BASF,
   Benzophenone-2 vendu sous le nom commercial « UVINUL D50 » par BASF
   Benzophenone-3 ou Oxybenzone, vendu sous le nom commercial « UVINUL M40 » par BASF,
   Benzophenone-4 vendu sous le nom commercial « UVINUL MS40 » par BASF,
   Benzophenone-5
   Benzophenone-6 vendu sous le nom commercial « Helisorb 11 » par Norquay
   Benzophenone-8 vendu sous le nom commercial « Spectra-Sorb UV-24 » par American Cyanamid
   Benzophenone-9 vendu sous le nom commercial« UVINUL DS-49» par BASF,
   Benzophenone-12
   2-(4-diethylamino-2-hydroxybenzoyl)-benzoate de n-hexyle
Dérivés du benzylidène camphre :
   3-Benzylidene camphor fabriqué sous le nom « MEXORYL SD» par CHIMEX,
   4-Methylbenzylidene camphor vendu sous le nom « EUSOLEX 6300 » par MERCK,
   Benzylidene Camphor Sulfonic Acid fabriqué sous le nom « MEXORYL SL» par CHIMEX,
   Camphor Benzalkonium Methosulfate fabriqué sous le nom « MEXORYL SO » par CHIMEX,
   -Terephthalylidene Dicamphor Sulfonic Acid fabriqué sous le nom « MEXORYL SX » par CHIMEX,
   Polyacrylamidomethyl Benzylidene Camphor fabriqué sous le nom « MEXORYL SW » par CHIMEX,
Dérivés du phenyl benzimidazole :
   Phenylbenzimidazole Sulfonic Acid vendu notamment sous le nom commercial « EUSOLEX 232 » par MERCK,
   Disodium Phenyl Dibenzimidazole Tetra-sulfonate, vendu sous le nom commercial commercial « NEO HELIOPAN AP » par Haarmann et REIMER,
Dérivés de la triazine :
   Anisotriazine vendu sous le nom commercial «TINOSORB S » par CIBA GEIGY,
   Ethylhexyl triazone vendu notamment sous le nom commercial «UVINUL T150 » par BASF,
   Diethylhexyl Butamido Triazone vendu sous le nom commercial « UVASORB HEB » par SIGMA 3V,
Dérivés du phenyl benzotriazole :
   Drometrizole Trisiloxane vendu sous le nom « Silatrizole » par RHODIA CHIMIE , Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,
Dérivés anthraniliaues :
   Menthyl anthranilate vendu sous le nom commercial commercial « NEO HELIOPAN MA » par Haarmann et REIMER,
Dérivés d'imidazolines :
   Ethylhexyl Dimethoxybenzylidene Dioxoimidazoline Propionate,
Dérivés du benzalmalonate :
   Polyorganosiloxanes à fonctions benzalmalonate comme le Polysilicone-15 vendu sous la dénomination commerciale « PARSOL SLX » par HOFFMANN LAROCHE
Dérivés de 4,4-diarylbutadiène :
   -1,1-dicarboxy (2,2'-diméthyl-propyl)-4,4-diphénylbutadiène
   et leurs mélanges.

Les filtres UV organiques plus particulièrement préférés sont choisis parmi les composés suivants :
Ethylhexyl Salicylate,
Butyl Methoxydibenzoylmethane,
Ethylhexyl Methoxycinnamate,
Octocrylene,
Phenylbenzimidazole Sulfonic Acid,
Terephthalylidene Dicamphor Sulfonic,.
Benzophenone-3,
Benzophenone-4,
Benzophenone-5,
2-(4-diethylamino-2-hydroxybenzoyl)-benzoate de n-hexyle
4-Methylbenzylidene camphor,
Disodium Phenyl Dibenzimidazole Tetra-sulfonate,
Anisotriazine,
Ethylhexyl triazone,
Diethylhexyl Butamido Triazone,
Methylene bis-Benzotriazolyl Tetramethylbutylphenol,
Drometrizole Trisiloxane,
Polysilicone-15,
1,1-dicarboxy (2,2'-diméthyl-propyl)-4,4-diphénylbutadiène,
et leurs mélanges.

Les agents photoprotecteurs inorganiques sont choisis parmi les pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium et leurs mélanges. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP 518 772 et EP 518 773.

Les agents photoprotecteurs sont généralement présents dans les compositions selon l'invention dans des proportions allant de 0,1 à 20% en poids par rapport au poids total de la composition, et de préférence allant de 0,2 à 15% en poids par rapport au poids total de la composition.

Bien entendu, l'homme de l'art veillera à choisir le ou les éventuels composés complémentaires cités ci-dessus et/ou leurs quantités de manière telle que les propriétés avantageuses attachées intrinsèquement à l'association conforme à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait ou sous la forme d'un gel ou d'un gel crème, sous la forme d'une lotion, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans-huile.

Lorsqu'il s'agit d'une émulsion, la phase aqueuse de celle-ci peut comprendre une dispersion vésiculaire non ionique préparée selon les procédés connus (Bangham, Standish and Watkins J. Mol. Biol. 13, 238 (1965), FR 2 315 991 et FR 2 416 008).

L'invention concerne également un procédé de traitement cosmétique pour bronzer et/ou brunir artificiellement la peau, caractérisé par le fait qu'il consiste à appliquer sur celle-ci une quantité efficace d'une composition cosmétique telle que définie ci-dessus.

L'invention concerne également l'utilisation d'au moins un dérivé ou un mélange de dérivés de la 1,7-bisphényl heptane-3,5-dione de formule (I) tel que défini précédemment dans le but d'améliorer la coloration et/ou la stabilité d'un agent autobronzant tels que ceux définis ci-dessus contenu dans une composition cosmétique destinée au bronzage et/ou brunissage artificiels de la peau.

Des exemples concrets, mais nullement limitatifs, illustrant l'invention, vont maintenant être donnés.

Dans la composition ci-dessous, les quantités sont exprimées en grammes.

| **Composition de l'invention** | **Quantité (grammes)** |
|---|---|
| Hydroxypropyl methyl cellulose | 2 |
| Glycérine | 5 |
| Propylène Glycol | 10 |
| Ethanol Absolu | 10 |
| Dihydroxyacetone | 4 |
| Mélange de tétrahydrocurcuminoïdes de formule (I) (Tetrahydrocurcuminoids CG de Sabinsa) | 1 |
| Eau déminéralisée qsp | 100g |

## Revendications

1. Composition cosmétique et/ou dermatologique, **caractérisée par le fait qu'**elle contient dans un support cosmétiquement acceptable :
(i) au moins un agent autobronzant, et
(ii) au moins un dérivé ou un mélange de dérivés de la 1,7-bisphényl heptane-3,5-dione de formule (I) suivante : 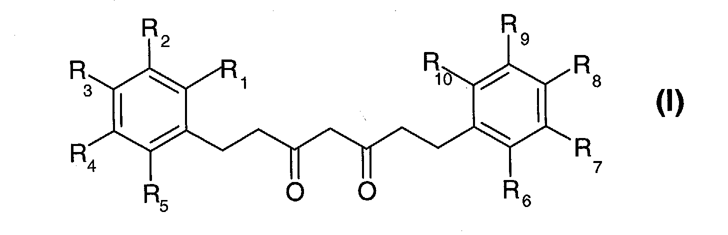
dans laquelle :
R₁ , R₂ , R₃, R₄ , R₅ , R₆, R₇, R₈, R₉, R₁₀, identiques ou différents, sont choisis parmi:
(i) un atome d'hydrogène ;
(ii) un radical alkyle linéaire ou ramifié en C₁-C₄ ;
(iii) un radical OR₁₁ dans lequel R₁₁ est choisi parmi un atome d'hydrogène, un radical alkyle linéaire ou ramifié en C₁-C₄, un radical PO (OX₁)(OX₂) ou un radical SO₂(OX₃) dans lesquels, X₁, X₂, et X₃, identiques ou différents, désignent un atome d'hydrogène ou un cation de métal alcalin ou NH₄⁺ , X₁ et X₂ pouvant également désigner ensemble un cation de métal divalent,
(iv) un radical acyle R₁₂CO dans lequel R₁₂ est choisi parmi des radicaux hydrocarbonés en C₁-C₃₀, qui sont linéaires ou ramifiés, saturés ou non saturés, hydroxylés ou non hydroxylés, carboxylés ou non carboxylés ;
(v) un radical glycosyle ou uronyle ;
lorsqu'un radical R₁ à R₁₀ désigne un reste OR₁₁, il peut également former avec le cycle aromatique auquel il est attaché et un radical adjacent un cycle contenant 5 ou 6 atomes ; étant entendu qu'au moins un des radicaux R₁ à R₁₀ désigne un reste OR₁₁.

2. Composition selon la revendication 1, où le ou les composés de formule (I) sont choisis dans le groupe constitué par :
- la 1,7-bis-(3-méthoxy-4-hydroxy phényl)heptane-3,5-dione) ou tétrahydrocurcumine (THC) de structure : 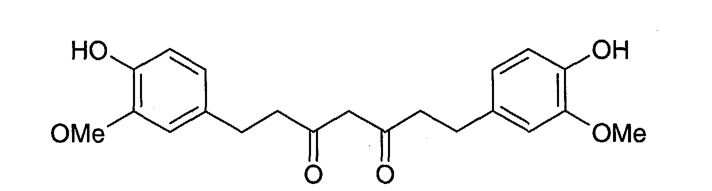
- la 1-(3-méthoxy-4-hydroxy phényl) 7-(4'-hydroxy phényl) heptane-3,5-dione ou tétrahydrodéméthoxycurcumine (THDC) de structure : 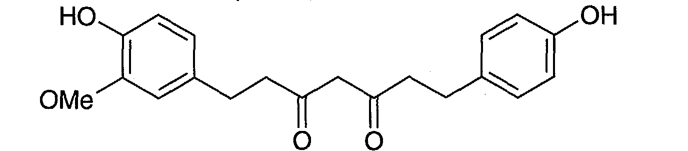
- la 1,7-bis-(4-hydroxy phényl) heptane-3,5-dione ou tétrahydrobis déméthoxycurcumine (THBDC) de structure : 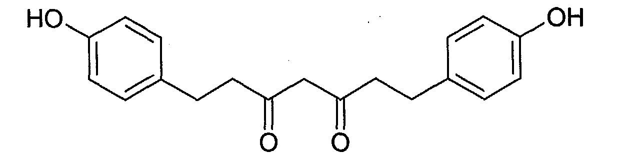
- la 1,7-bis(3,4-dihydroxyphenyl) heptane-3,5-dione de structure : 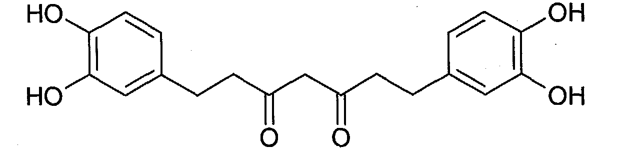
ainsi que leurs mélanges.

3. Composition selon la revendication 2, **caractérisée par le fait qu'**elle contient, comme seul composé de formule (I), la 1,7-bis-(3-méthoxy-4-hydroxyphényl)heptane-3,5-dione) ou tétrahydrocurcumine (THC).

4. Composition selon la revendication 1 ou 2, **caractérisée par le fait qu'**elle contient un mélange de composés de formule (I) constitué :
de tétrahydrocurcumine ;
de tétrahydro-déméthoxycurcumine et
de tétrahydro-bis-déméthoxycurcumine.

5. Composition selon la revendication 4, **caractérisée par le fait qu'**elle contient un mélange de composés de formule (I) constitué :
- de 70 à 95% en poids de tétrahydrocurcumine ;
- de 4 à 25% en poids de tétrahydro-déméthoxycurcumine et
- de 0,5 à 10 % en poids de tétrahydro-bis-déméthoxycurcumine.

6. Composition selon la revendication 5, caractérisée qu'elle contient un mélange de composés de formule (I) constitué :
- de 75 à 90% en poids de tétrahydrocurcumine ;
- de 8 à 20% en poids de tétrahydro-déméthoxycurcumine et
- de 1 à 5 % en poids de tétrahydro-bis-déméthoxycurcumine.

7. Composition selon l'une quelconque des revendications 1 à 6, **caractérisée par le fait que** le ou les composés de formule (I) représentent de 0,001 à 10% en poids du poids total de la composition cosmétique, plus particulièrement de 0,01 à 5%, et encore plus préférentiellement de 0,1 à 5% en poids environ de ce poids.

8. Composition selon l'une des revendications 1 à 7, telle que l'agent autobronzant est un composé mono ou polycarbonylé.

9. Composition selon la revendication 8, telle que l'agent autobronzant est choisi dans le groupe formé par l'isatine, l'alloxane, la ninhydrine, le glycéraldéhyde, l'aldéhyde mésotartrique, la glutaraldéhyde, l'érythrulose, les dérivés de pirazoline -4,5-dione, la dihydroxyacétone (DHA), les dérivés de 4,4-dihydroxypirazoline-5-diones.

10. Composition selon la revendication 13, telle que l'agent autobronzant est la DHA.

11. Composition selon l'une quelconque des revendications 1 à 10, telle que la concentration en agent autobronzant varie de 0,1 à 10% en poids, par rapport au poids total de la composition.

12. Composition selon l'une des revendications 1 à 11, **caractérisée par le fait qu'**elle comporte au moins un colorant direct, synthétique ou naturel, et/ou au moins un dérivé indolique.

13. Composition selon l'une des revendications 1 à 12, telle qu'elle comprend un agent de coloration additionnel choisi parmi les extraits de bois rouges "insolubles" du genre Pterocarpus et du genre Baphia .

14. Composition selon l'une des revendications 1 à 13, telle qu'elle comprend un agent de coloration additionnel choisi parmi les nanopigments d'oxyde de fer dont la taille moyenne des particules élémentaires est inférieure à 100 nm.

15. Composition selon l'une quelconque des revendications 1 à 14, **caractérisée par le fait qu'**elle comporte au moins un adjuvant cosmétique parmi les corps gras, les solvants organiques, les épaississants ioniques ou non ioniques, les adoucissants, les humectants, les opacifiants, les stabilisants, les émollients, les silicones, les agents anti-mousse, les agents répulsifs contre les insectes, les parfums, les conservateurs, les tensioactifs anioniques, cationiques, non-ioniques, zwitterioniques ou amphotères, les antagonistes de substance P, les antagonistes de substance CGRP, les charges, les polymères, les propulseurs, les agents alcalinisants ou acidifiants ou tout autre ingrédient habituellement utilisé dans le domaine cosmétique et/ou dermatologique.

16. Composition selon l'une quelconque des revendications 1 à 15, **caractérisée par le fait qu'**elle comporte en plus en outre au moins un agent photoprotecteur organique et/ou au moins un agent photoprotecteur inorganique actifs dans l'UVA et/ou dans l'UVB.

17. Composition selon la revendication 16, telle que l'agent photoprotecteur organique est choisis parmi les anthranilates ; les dérivés cinnamiques ; les dérivés de dibenzoylméthane ; les dérivés salicyliques, les dérivés du camphre ; les dérivés de triazine ; les dérivés de la benzophénone ; les dérivés de β,β'-diphénylacrylate ; les dérivés de benzotriazole ; les dérivés de benzimidazole ; les imadazolines ; les dérivés bis-benzoazolyle ; les dérivés de l'acide p-aminobenzoïque (PABA) ; les dérivés de méthylène bis-(hydroxyphényl benzotriazole) ; les polymères filtres et silicones filtres ; les dimères dérivés d'α-alkylstyrène ; les dérivés de 4,4-diarylbutadiène ; les hydroxybenzophénones aminées et leurs mélanges.

18. Composition selon la revendication 17, telle que l'agent photoprotecteur organique est choisi parmi :
Ethylhexyl Salicylate,
Butyl Methoxydibenzoylmethane,
Ethylhexyl Methoxycinnamate,
Octocrylene,
Phenylbenzimidazole Sulfonic Acid,
Terephthalylidene Dicamphor Sulfonic,.
Benzophenone-3,
Benzophenone-4,
Benzophenone-5,
2-(4-diethylamino-2-hydroxybenzoyl)-benzoate de n-hexyle
4-Methylbenzylidene camphor,
Disodium Phenyl Dibenzimidazole Tetra-sulfonate,
Anisotriazine,
Ethylhexyl triazone,
Diethylhexyl Butamido Triazone,
Methylene bis-Benzotriazolyl Tetramethylbutylphenol,
Drometrizole Trisiloxane,
Polysilicone-15
1,1-dicarboxy (2,2'-diméthyl-propyl)-4,4-diphénylbutadiène
et leurs mélanges.

19. Composition selon la revendication 20, telle que l'agent photoprotecteur organique est choisi parmi les pigments ou les nanopigments d'oxydes métalliques enrobés ou non.

20. Composition selon la revendication 19, telle que l'agent photoprotecteur organique est choisi parmi les nanopigments enrobés ou non, d'oxyde de titane, de fer, de zinc, de zirconium ou de cérium et leurs mélanges.

21. Composition selon l'une quelconque des revendications 20 à 24 telle que l'agent photoprotecteur organique est présent dans les compositions dans des proportions allant de 0,1 à 20% en poids par rapport au poids total de la composition, et de préférence allant de 0,2 à 15% en poids par rapport au poids total de la composition.

22. Composition selon l'une des revendications précédentes, telle qu'elle se présente sous la forme d'une dispersion vésiculaire non ionique, d'une émulsion, en particulier d'une émulsion de type eau-dans huile, de type huile-dans-eau, d'une crème, ou d'une émulsion triple (E/H/E ou H/E/H), d'un lait, d'un gel, d'un gel crème, d'une suspension, d'une dispersion, d'une mousse ou d'un spray.

23. Utilisation d'une composition selon la revendication 22 à titre de composition cosmétique destinée au bronzage ou au brunissement de la peau.

24. Procédé cosmétique de bronzage ou brunissement de la peau **caractérisé en ce qu'**il consiste à appliquer sur la peau une quantité efficace d'une composition cosmétique selon l'une des revendications 1 à 23.

25. Utilisation d'au moins un dérivé ou un mélange de dérivés de la 1,7-bisphényl heptane-3,5-dione de formule (I) tel que défini dans l'une quelconque des revendications 1 à 6 dans une composition pour le bronzage et/ou le brunissage artificiels de la peau contenant au moins un agent autobronzant dans le but d'améliorer la coloration et/ou la stabilité dudit agent autobronzant.
